Europäisches Patentamt

European Patent Office

Office européen des brevets

(19)

(11) Numéro de publication: **0 068 961**
**A2**

(12) **DEMANDE DE BREVET EUROPEEN**

(21) Numéro de dépôt: 82401068.0

(22) Date de dépôt: **11.06.82**

(51) Int. Cl.³: **A 61 F 7/00**, G 10 K 11/34

(30) Priorité: **26.06.81 FR 8112612**
**19.03.82 FR 8204747**

(43) Date de publication de la demande: **05.01.83**
**Bulletin 83/1**

(84) Etats contractants désignés: **DE GB IT NL**

(71) Demandeur: **THOMSON-CSF, 173, Boulevard Haussmann, F-75379 Paris Cedex 08 (FR)**

(72) Inventeur: **Do Huu, Jean-Paul, THOMSON-CSF SCPI 173, bld Haussmann, F-75379 Paris Cedex 08 (FR)**
Inventeur: **Hartemann, Pierre, THOMSON-CSF SCPI 173, bld Haussmann, F-75379 Paris Cedex 08 (FR)**

(74) Mandataire: **Wang, Pierre et al, THOMSON-CSF SCPI 173, Bld Haussmann, F-75379 Paris Cedex 08 (FR)**

(54) Dispositif d'échauffement localisé de tissus biologiques.

(57) L'invention se rapporte aux dispositifs de traitement hyperthermique des tissus biologiques par un apport de chaleur localisé au moyen d'un rayonnement focalisé.

L'invention a pour objet un dispositif d'échauffement localisé à ultrasons dans lequel l'irradiateur est une plaque piézoélectrique (1) subdivisée en zones rayonnantes annulaires d'égales largeurs par un ensemble de sillons circulaires concentriques (4).

L'invention s'applique notamment au traitement des tumeurs par ultrasons.

0068961

DISPOSITIF D'ECHAUFFEMENT LOCALISE DE TISSUS BIOLOGIQUES

La présente invention se rapporte à l'utilisation de la chaleur comme agent thérapeutique susceptible d'être appliqué localement à des tissus biologiques profonds. On sait que l'échauffement d'un tissu biologique nécessite un apport d'énergie qui ne peut être fourni par simple conduction thermique à travers le corps. Il est donc nécessaire de transporter l'énergie vers la zone à échauffer au moyen d'un rayonnement qui converge vers cette zone. Ainsi, on évite d'échauffer les tissus traversés par le rayonnement entre la surface émissive et la zone de focalisation. L'ultilisation d'un rayonnement électromagnétique de très haute fréquence permet d'obtenir une bonne focalisation avec un applicateur de taille raisonnable, mais la profondeur de pénétration est insuffisante pour traiter par hyperthermie des tissus profonds. Des rayonnements électromagnétiques de fréquence plus basse peuvent pénétrer plus profondément dans le corps humain, mais la partie soumise à l'échauffement comprend tous les tissus situés dans la région irradiée.

On sait que les tissus biologiques renferment une grande quantité d'eau et qu'ils sont perméables aux rayonnements ultrasonores de faible longueur d'onde. On peut donc fournir un apport de chaleur localisé en profondeur à l'aide d'un émetteur d'ultrasons de taille réduite qu'il suffit d'appliquer contre le patient via une poche d'eau. Ce mode de traitement ne nécessite pas l'implantation d'électrodes pour localiser l'échauffement, ce qui lui confère un caractère non traumatisant.

L'applicateur peut revêtir une forme particulièrement simple et son excitation au moyen de signaux électriques faciles à moduler en phase et en amplitude apporte une grande souplesse dans le traitement par hyperthermie.

L'invention a pour objet un dispositif d'échauffement localisé de tissus biologiques par projection de rayonnement ultrasonore au moyen d'un transducteur électromécanique associé à un générateur électrique d'excitation ayant une fréquence d'oscillation prédéterminée, caractérisé en ce que ledit transducteur est du type demi-onde et comporte une plaque plane de matériau piézoélectrique dont une première face comporte un réseau de sillons annulaires concentriques ayant un pas régulier voisin de la longueur d'onde dans l'eau dudit rayonnement ; les zones rayonnantes annulaires de ladite plaque encadrées par lesdits sillons étant munies d'électrodes en contact avec lesdites faces ; ledit générateur électrique comportant un ensemble de moyens déphaseurs réglables destiné à alimenter au moins partiellement lesdites électrodes par des tensions dont les phases sont ajustables pour focaliser ledit rayonnement au sein des tissus biologiques couplés acoustiquement avec ladite face.

L'invention sera mieux comprise au moyen de la description ci-après et des figures annexées parmi lesquelles :

- la figure 1 est une vue isométrique partielle d'un dispositif selon l'invention ;

- la figure 2 est une coupe méridienne du dispositif applicateur de la figure 1 ;

- les figures 3 à 5 sont des figures explicatives ;

- la figure 6 représente une variante de réalisation du dispositif applicateur de la figure 1 ;

- la figure 7 illustre une autre variante de réalisation du dispositif de la figure 1.

- la figure 8 est une vue isométrique d'un dispositif d'échauffement selon l'invention ;

- la figure 9 illustre une première variante de réalisation du mode de projection selon la figure 8 ;

- la figure 10 illustre une seconde variante de réalisation du mode de projection selon la figure 8.

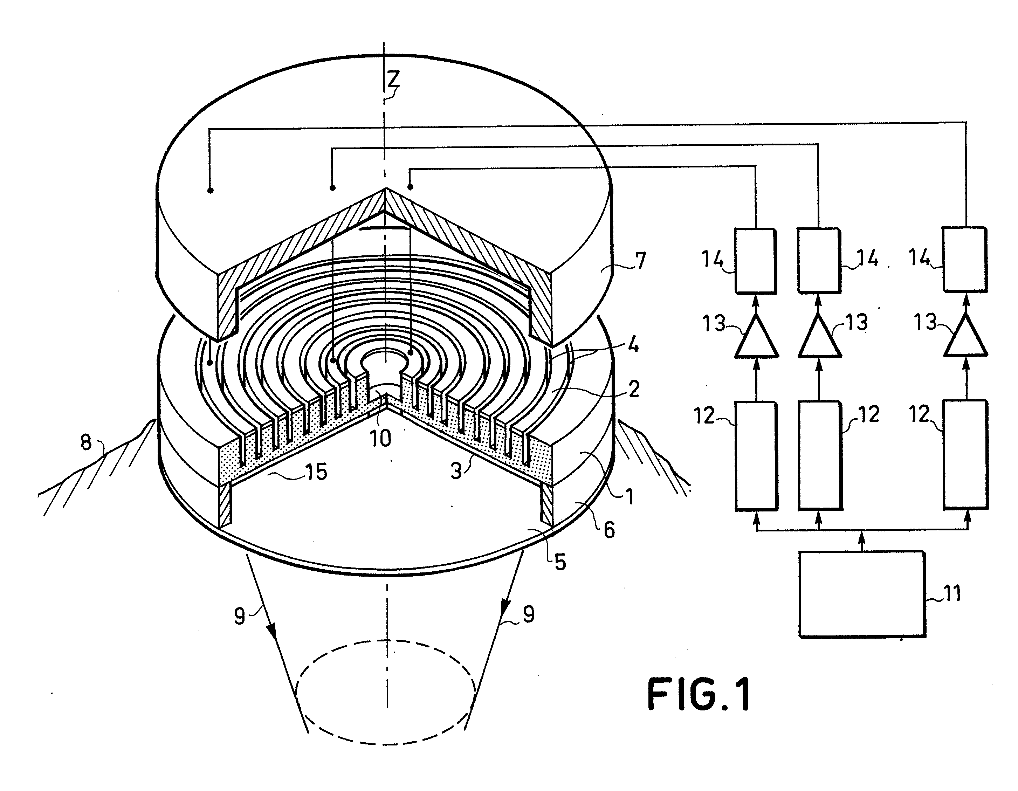

Sur la figure 1, on peut voir un dispositif à rayonnement ultrasonore permettant d'obtenir un échauffement localisé de tissus biologiques. Le dispositif se compose de moyens d'excitation électriques représentés à droite sur la figure 1 et d'un applicateur dont la partie gauche de la figure 1 donne une vue partielle éclatée. L'applicateur est un transducteur électromécanique que l'on applique à la surface 8 d'un corps afin de rayonner vers l'intérieur un faisceau focalisé 9 d'ultrasons. L'émission ultrasonore dans les tissus biologiques sous-jacents à la surface 8 est dûe à une plaquette de matériau piézoélectrique 1 taillée en forme de disque. La distance e séparant les faces circulaires parallèles de la plaquette 1 est choisie de manière que le transducteur fonctionne en demi-onde à la fréquence d'excitation $f_o$. on a donc : $e = \dfrac{v}{2f_o}$, où v est la vitesse de phase des ondes ultrasonores dans le matériau piézoélectrique. Pour un matériau piézoélectrique tel qu'une céramique, en tablant sur une vitesse de propagation $v = 4000\ m.s^{-1}$ et sur une émission ultrasonore à la fréquence de 1 MHz, on trouve une épaisseur de plaquette de l'ordre de 2 mm. Selon une caractéristique de l'invention, le rayonnement ultrasonore focalisé 9 est rayonné par la face inférieure plane de la plaquette 1 qui est recouverte d'une métallisation 3. Selon une autre caractéristique de l'invention, la face rayonnante lisse 3 est couplée par un liquide ayant l'impédance acoustique des tissus biologiques à insonifier, à une membrane 5 qui vient en contact avec la surface 8. Un cadre circulaire 6 fixé à la plaquette 1 sert de support marginal à la membrane 5 qui est avantageusement constituée par un film souple. Une cavité étanche 15 est ainsi formée devant la face rayonnante lisse de la plaquette 1. Le liquide de remplissage de cette cavité 15 peut être avantageusement de l'eau et la poche ainsi constituée évite la distorsion des fronts d'ondes ultrasonores rayonnés par la plaquette 1. La poche d'eau joue aussi le rôle de dissipateur thermique car il faut évacuer la chaleur dégagée au sein de la plaquette. On peut prévoir une circulation du liquide en liaison avec un radiateur externe raccordé par des conduits non représentés sur la figure 1. Compte tenu du mode de vibration en demi-onde de la plaquette 1, il faut que l'impédance acoustique couplée à sa face supérieure 2 soit négligeable vis-à-vis de l'impédance acoustique de la céramique piézoélectrique. Cette faible impédance acoustique est avantageusement obtenue en coiffant la face supérieure 2 de la plaquette 1 avec un capot 7 qui renferme une couche d'air.

Pour émettre un rayonnement ultrasonore focalisé à partir d'une face rayonnante plane, il faut faire en sorte que plusieurs zones annulaires concentriques de celle-ci rayonnent des ondes décalées en phase selon une loi introduisant des déphasages fonction du rayon moyen de ces zones. A cet effet, la plaquette 1 est creusée de sillons annulaires 4 sur une profondeur pouvant atteindre 90 % de l'épaisseur e de la plaquette 1. Chaque anneau de matériau piézoélectrique compris entre deux sillons 4 successifs constitue une source ultrasonore élémentaire convenablement découplée de ses voisines. Une métallisation annulaire 2 coopère avec la métallisation 3 pour créer une excitation par champ électrique de chaque zone annulaire émissive. L'alimentation des sources ultrasonores élémentaires est assurée par un générateur de tension alternative 11 qui délivre à un ensemble de déphaseurs réglables 12 une tension alternative de fréquence $f_o$. Les tensions déphasées fournies par les déphaseurs 12 sont amplifiées par un ensemble d'amplificateurs à gain réglable 13. Un ensemble de circuits correcteurs et adaptateurs d'impédance (14) connectés entre chacune des métallisations 2 et la métallisation 3 assurent la compensation de la partie réactive de l'impédance électrique de chaque source piézoélectrique élémentaire et le cas échéant l'adaptation d'impédance. Les bornes de sortie des amplificateurs 13 sont donc chargées par la partie résistive de cette impédance électrique, ce qui assure le meilleur transfert de puissance d'excitation.

Sur la figure 1, la métallisation 3 fait office de contre-électrode reliée à la masse électrique commune des circuits correcteurs 14 et des amplificateurs de puissance 13.

Pour que la focalisation du rayonnement ultrasonore 9 se fasse sans lobes secondaires, le pas des sillons 4 ne doit pas s'écarter sensiblement de la longueur d'onde dans l'eau du rayonnement ultrasonore. La vitesse de propagation dans l'eau des ondes ultrasonores étant voisine de 1500 m/sec, on voit que le pas p est de l'ordre de 1,5 mm pour la fréquence d'émission de 1 MHz. En général, on a intérêt à réduire au minimum le nombre de sources élémentaires pour simplifier la réalisation des moyens d'excitation électrique ce qui amène à choisir le pas le plus grand qui conduise à l'élimination des lobes secondaires. La largeur radiale des sources annulaires élémentaires est choisie aussi proche que possible du pas p pour obtenir la plus grande surface rayonnante possible. En tenant compte des indications qui précèdent, on voit que pour la céramique piézoélectrique le rapport l/e sera voisin de 0,75. Pratiquement, on peut choisir le rapport l/e pour éviter les modes de résonances multiples pouvant apparaître avec la configuration morcelée de la plaquette 1. En effet, les transducteurs élémentaires peuvent ne pas résonner selon le seul mode d'épaisseur et il faut choisir l'optimum en tenant compte aussi du coefficient de couplage électromécanique. Ceci amène à choisir le rapport l/e entre les limites 0,5 et 1.

Pour que la fréquence centrale de fonctionnement de chaque source élémentaire corresponde aussi exactement que possible avec la fréquence d'excitation $f_o$, on adopte une largeur l constante pour tous les anneaux rayonnants. Ce type de transducteur diffère donc sensiblement d'un réseau émetteur conçu en accord avec la théorie des zones de Fresnel car dans ce dernier cas, la largeur radiale des zones émissives varient du centre vers la périphérie du réseau.

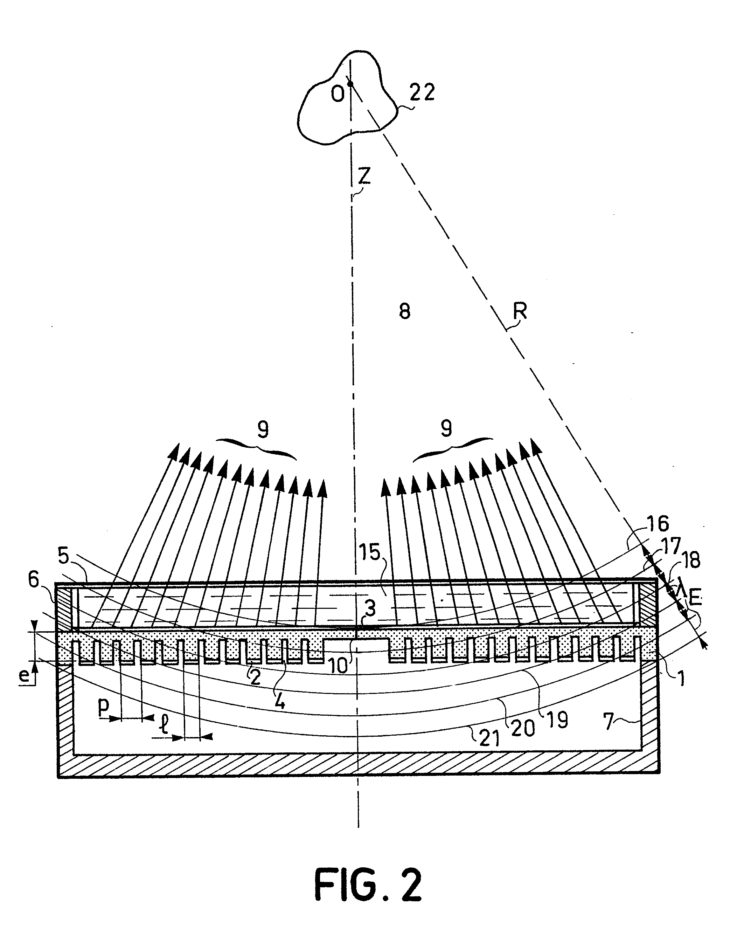

La coupe méridienne illustrée sur la figure 2 correspond à l'applicateur de la figure 1. Les mêmes références désignent les mêmes éléments et plus particulièrement on voit au centre de la plaquette un évidement central 10 qui de préférence ne participe pas à l'émission ultrasonore bien que le fonctionnement avec transducteur central soit compris dans l'invention. Sur la coupe de la figure 2, on a représenté les tissus biologiques 8 insonifiés par les rayonnements 9 qui convergent au point 0 d'une zone 22 à échauffer sélectivement. Le point de focalisation est supposé situé sur l'axe de révolution Z de la structure émettrice. Pour illustrer les déphasages qu'il faut respecter pour émettre un faisceau ultrasonore focalisé en 0, on a tracé sur la figure 2 plusieurs fronts d'ondes sphériques équiphases 16, 17, 18, 19, 20 et 21. Ces fronts d'ondes 16 à 21 sont distants de $\lambda_E$, longueur d'onde dans le milieu 8 des ondes ultrasonosres. Ils ont un centre de phase commun en 0. Le front d'onde 16 de rayon de courbure R tangente la face émissive 3 du transducteur piézoélectrique. Le front d'onde 17 représente un retard de phase de 2 π radiants, le front d'onde 18 un autre retard de phase de 2 π et ainsi de suite. En prenant pour référence de phase le premier anneau rayonnant en partant du centre de la face 3, on peut lire sur la figure 2 les avances de phase moyennes qu'il faut imposer aux tensions d'excitation à appliquer aux électrodes 2 pour obtenir la convergence en O. On voit que le septième anneau à partir du centre peut être excité en phase avec le premier de même qu'avec le dixième. Plus on s'écarte du centre, plus l'avance de phase doit croître rapidement. En fait, on fait varier le déphasage par bonds pour obtenir par gradins une approximation du front d'onde sphérique. Cette approximation est d'autant moins bonne que l'on s'écarte du centre et que la largeur l est grande par rapport à $\lambda_E$. On veillera à ne pas dépasser une erreur de phase de $\pi/2$ radian sur la largeur de l'anneau, ce qui en fonction de l'ouverture du faisceau déterminera la largeur l de chaque anneau rayonnant. Le film souple 5 peut se mouler à la surface du corps 8 sans donner lieu à une réfraction perturbatrice à l'interface entre le liquide 15 et les tissus biologiques, car les vitesses acoustiques sont égales. Le film souple 5 peut être avantageusement un film polymère mince qui évite une rupture d'impédance susceptible de donner lieu à une réflexion du rayonnement 9.

Le transducteur annulaire multiple des figures 1 et 2 peut être excité de différentes façons. On peut par exemple prévoir une puissance acoustique par unité de surface de valeur uniforme. Ceci est obtenu aisément dans le cas d'anneaux émetteurs de largeur l constante, car l'impédance ramenée entre la métallisation 3 et la métallisation 2 est inversement proportionnelle au rayon moyen de l'anneau. Comme la surface rayonnante est proportionnelle au rayon, il suffira d'appliquer la même tension d'excitation à tous les anneaux. Bien entendu, pour donner à l'insonification une répartition particulière au foyer, on peut faire appel à la technique d'apodisation. Les ajustements de tension d'excitation s'obtiennent en jouant sur le gain des amplificateurs.

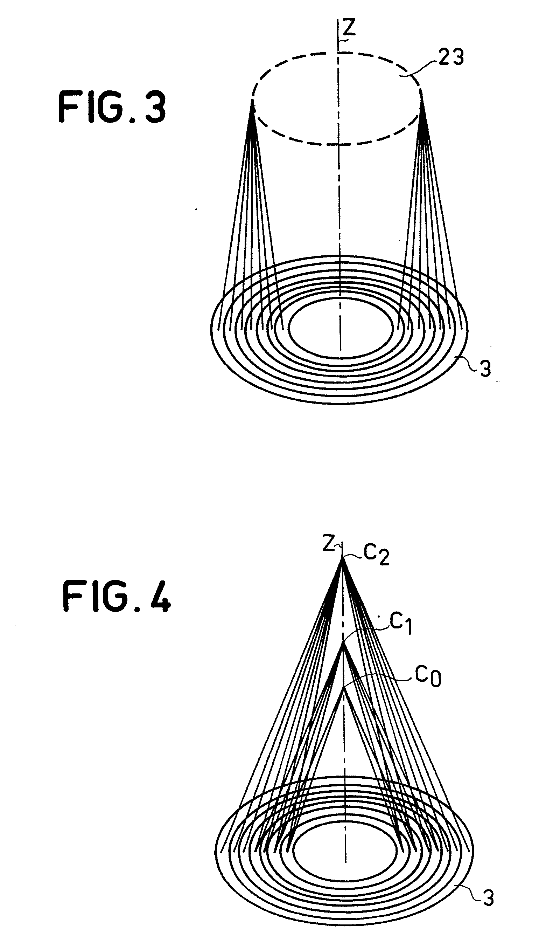

En ce qui concerne les ajustements de phase, pour obtenir une focalisation ponctuelle, on a vu que les ondes ultrasonores rayonnées par les sources annulaires doivent parvenir en phase au foyer désiré. Sur la figure 4, cette focalisation est illustrée pour trois foyers $C_0$, $C_1$ et $C_2$ avec conservation de l'ouverture angulaire du faisceau. On obtient aisément ce résultat par une adaptation de la loi de phase et par l'utilisation d'un nombre plus ou moins important d'anneaux rayonnant. Sur la figure 4, la focalisation en $C_0$ fait appel à deux anneaux rayonnants centraux mais la focalisation en $C_2$ met en oeuvre tous les anneaux de la face émissive 3. Pour obtenir une ouverture angulaire constante le nombre d'anneaux varie au prorata de la distance de projection.

Cette technique permet de conserver des dimensions inchangées à la zone d'échauffement tant en largeur qu'en profondeur. Le nombre d'anneaux utilisés étant alors proportionnel à la profondeur, on réalise ainsi une compensation de la perte de puissance dûe à l'atténuation de propagation.

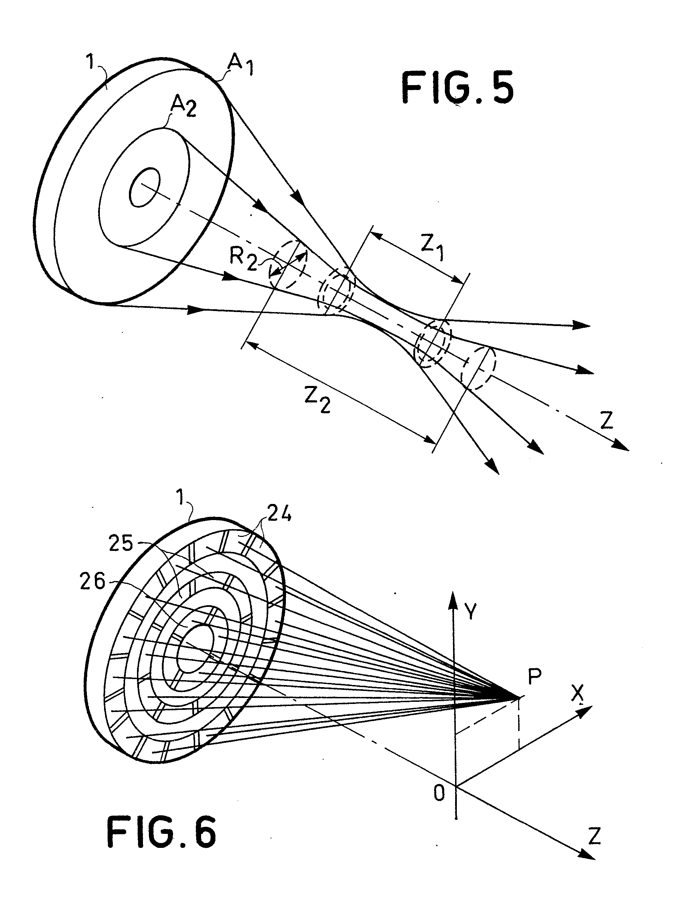

Le fonctionnement avec un nombre plus ou moins grand d'éléments rayonnants permet de faire varier l'ouverture angulaire d'un faisceau ultrasonore focalisé à une distance prédéterminée. Sur la figure 5, on a illustré une plaquette piézoélectrique de face rayonnante $A_1$. Lorsque tous les transducteurs annulaires qui composent cette face sont excités, la focalisation intervient suivant l'axe Z de projection normale. Transversalement la répartition de l'intensité ultrasonore est régie par les lois de la diffraction d'une pupille annulaire. Longitudinalement la zone d'échauffement a une profondeur $Z_1$ correspondant à la profondeur de champ. En réduisant la face active à la zone $A_2$, on voit que la profondeur $Z_2$ et la largeur $R_2$ de la zone d'échauffement ont augmenté pour la même profondeur de focalisation.

Il est utile de rappeler les formules relatives à une pupille circulaire. La largeur à - 3 dB d'un faisceau focalisé est environ égale à $\frac{\lambda .F}{D}$ où $\lambda$ est la longueur d'onde, F la distance focale et D le diamètre de la surface émissive du rayonnement ultrasonore. La profondeur de champ à - 3 dB vaut sensiblement $7,14 \; \lambda (\frac{F}{D})^2$.

Une autre façon de focaliser le rayonnement ultrasonore est représentée sur la figure 3. Elle consiste à exciter les anneaux de la face rayonnante 3 avec une loi de phase amenant les ondes émises à être en concordance de phase sur une ligne focale circulaire 23. Cette technique permet d'élargir la zone chauffée où de chauffer uniquement le pourtour d'une tumeur.

D'après ce qui précède, on voit que le dispositif d'échauffement sélectif par ultrasons dont la structure et le fonctionnement sont illustrés par les figures 1 à 5 possède une grande souplesse de réglage tout en utilisant une surface rayonnante plane comme émetteur ultrasonore. Le réglage en amplitude et l'apodisation des sources se règle facilement au niveau des amplificateurs de puissance 13 qui possèdent une commande de gain. Les conditions de focalisation sur l'axe Z ou autour de l'axe Z se règlent au niveau des déphaseurs 12 qui peuvent être à titre d'exemple non limitatif des déphaseurs RC conservant l'amplitude et commandables électriquement par variation du produit RC. L'échauffement localisé depend comme on l'a vu des caractéristiques de phase et d'amplitude des tensions d'excitation appliquées à un nombre plus ou moins grand d'émetteurs annulaires élémentaires. Le générateur 11 peut fournir une tension alternative sinusoïdale ou une succession de trains d'ondes plus ou moins rapprochés ce qui ajoute une autre possibilité de réglage de l'échauffement. On peut également par commande programmée des moyens d'excitation réaliser un balayage de zone en profondeur et en largeur pour étaler l'échauffement. Enfin, il est possible d'utiliser plusieurs applicateurs émettant des rayonnements cohérents et convergents pour améliorer la localisation de l'échauffement.

La figure 6 illustre une autre possibilité offerte par le dispositif conforme à l'invention. En effet, chaque anneau rayonnant de la plaquette 1 peut être subdivisé en secteurs angulaires 24, 25 et 26 grace à des métallisations 2 segmentées qui peuvent coopérer le cas échéant avec des sillons agencés radialement. La subdivision des anneaux augmente du centre vers la périphérie comme le montre la figure 6 afin de maintenir la réponse en fréquence des émetteurs piézoélectriques élémentaires bien centrée sur la fréquence d'émission choisie. Les émetteurs élémentaires 24, 25, 26 sont reliés à des amplificateurs 13 via des circuits adaptateurs d'impédance 14. Un nombre plus important de déphaseurs 12 permet non seulement de créer une loi de déphasage destinée à permettre la focalisation de l'émission en un point P du plan XOY mais cette loi peut comprendre une dsitribution de déphasage dont l'ampleur détermine les coordonnées du point P dans le plan XY. Il devient donc possible d'adresser n'importe quel point du volume repéré par le système de coordonnées XYZ et si on le désire de faire évoluer dans le temps cet adressage. A cette possibilité s'ajoute bien entendu la modulation en amplitude ou en impulsion du générateur 11 qui permet un dosage supplémentaire de l'échauffement.

La technique d'échauffement par ultrasons qui vient d'être décrite a montré qu'elle peut ne pas utiliser comme émetteur chauffant la zone circulaire centrale de la plaquette piézoélectrique 1. On a donc la possibilité d'utiliser cette zone pour y loger un ensemble de moyens d'émission réception d'ultrasons de plus courte longueur d'onde afin de visualiser les tissus biologiques pendant le traitement par hyperthermie.

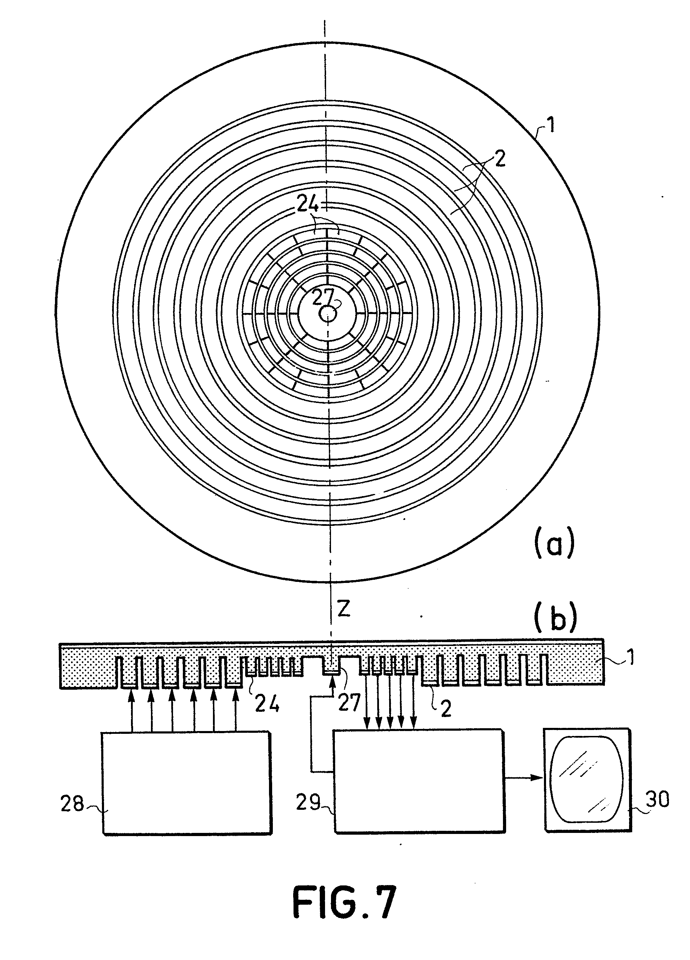

La figure 7 illustre cette possibilité. Une vue de dessous est visible en (a) et une coupe méridienne apparaît en (b). On retrouve comme aux figures 1 et 2 une plaquette piézoélectrique 1 portant un ensemble concentrique de métallisations annulaires 2 séparées par des sillons équidistants. Les métallisations 2 sont reliées à un ensemble d'excitation 28 qui regroupe les circuits électriques de la figure 1 et leurs moyens de commande. On est donc en présence d'un système d'échauffement par ultrasons du type décrit précédemment. Ce système est par exemple complété dans la partie centrale de la plaquette 1 par un système ultrasonore de visualisation dont les organes transducteurs 24 et 27 bénéficient de la même technologie à ceci près qu'ils ont une taille réduite pour fonctionner à plus haute fréquence et avec une puissance réduite. Cependant, on peut prévoir un ensemble transducteur d'imagerie entièrement réalisé dans une autre plaquette piézoélectrique. Plus précisément, et à titre d'exemple non limitatif, un élément transducteur central muni d'une électrode d'excitation 27 émet un rayonnement ultrasonore, par exemple à 3 MHz qui insonifie le champ à l'intérieur duquel on veut visualiser les tissus biologiques. Un ensemble électrique 29 fournit au transducteur central 27 des impulsions ultrasonores donnant naissance à des échos captés par le réseau de récepteurs sectoraux ou non 24. L'ensemble 29 reçoit en retour par les électrodes 24 un ensemble de signaux qui sont traités en fonction de l'amplitude de la phase et du temps de vol pour former sur un moniteur 30 une image visible du champ exploré. Il est superflu de donner plus de détails concernant la technique de visualisation car elle est abandamment décrite dans le domaine du sonar et de l'échographie. Par contre, il faut signaler que l'échauffement a une influence sur la vitesse de propagation des ultrasons qui peut atteindre une variation de 0,1 % par degré centigrade. En effectuant une mesure fine du temps de vol, on peut déduire une information relative au contrôle de l'échauffement produit. On peut également accéder à la mesure de l'échauffement produit en considérant la dérive en température du coefficient de réflexion d'une tumeur à soigner.

Pour terminer, on peut donner quelques indications chiffrées relatives à une réalisation typique d'un dispositif d'hyperthermie acoustique.

L'émission ultrasonore a lieu à une fréquence de 400 kHz avec une puissance acoustique de 50 W. L'irradiateur est constitué par une plaquette de céramique piézoélectrique d'un diamètre de 100 mm. Cette plaquette comporte quatorze anneaux émetteurs concentriques. La focalisation peut être assurée à des profondeurs comprises entre 20 mm et 120 mm de manière à échauffer un volume de quelques $cm^3$. A cette fréquence, l'absorption des ondes ultrasonores par les tissus biologiques est de l'ordre de 0,5 dB/cm ce qui assure une bonne pénétration à condition de ne rencontrer ni os, ni poche d'air.

La description qui précède se rapporte aux dispositifs d'échauffement localisé de tissus biologiques. Ces dispositifs sont basés sur la projection d'un rayonnement ultrasonore au moyen d'un applicateur muni d'un transducteur électromécanique qui irradie sélectivement la région à laquelle s'applique le traitement par hyperthermie. La région à traiter est sélectionnée par un processus de focalisation qui met en oeuvre un ensemble d'éléments rayonnants concentriques associés à des moyens déphaseurs. Les tissus biologiques situés en amont et en aval du foyer où se rencontre le rayonnement ultrasonore subissent un échauffement de moins en moins important lorsqu'on s'éloigne du foyer. Le problème qui se pose est d'adapter le dosage du rayonnement au foyer en fonction de la forme des volumes à échauffer et en tenant compte des pertes de chaleur provoquées par la vascularisation et la conduction thermique.

Les moyens d'irradiation précédemment décrits comprennent un disque de matériau piézoélectrique subdivisé en éléments rayonnants concentriques demi-onde par des tranchées annulaires pratiquées dans la face arrière à intervalles réguliers, du centre vers la périphérie du disque. En excitant électriquement un nombre plus ou moins important d'éléments rayonnants avec des signaux alternatifs convenablement déphasés, il est possible de projeter vers un foyer ponctuel ou annulaire un rayonnement ultrasonore assurant un échauffement localisé. Pour uniformiser l'échauffement dans une région traitée, il a été suggéré qu'en modifiant la loi de phase qui fixe l'emplacement du foyer, on peut appliquer successivement l'échauffement à plusieurs sites d'une même région à traiter. La modification d'une loi de phase est relativement complexe à mettre en oeuvre, aussi est-il souvent préférable d'utiliser plusieurs foyers irradiés simultanément lorsqu'on envisage de traiter une région relativement étendue ou plusieurs zones malades d'un organe volumineux. Dans ce cas, on peut faire appel à plusieurs applicateurs cités à la même fréquence pour cerner un volume tumoral ou un ensemble de tumeurs.

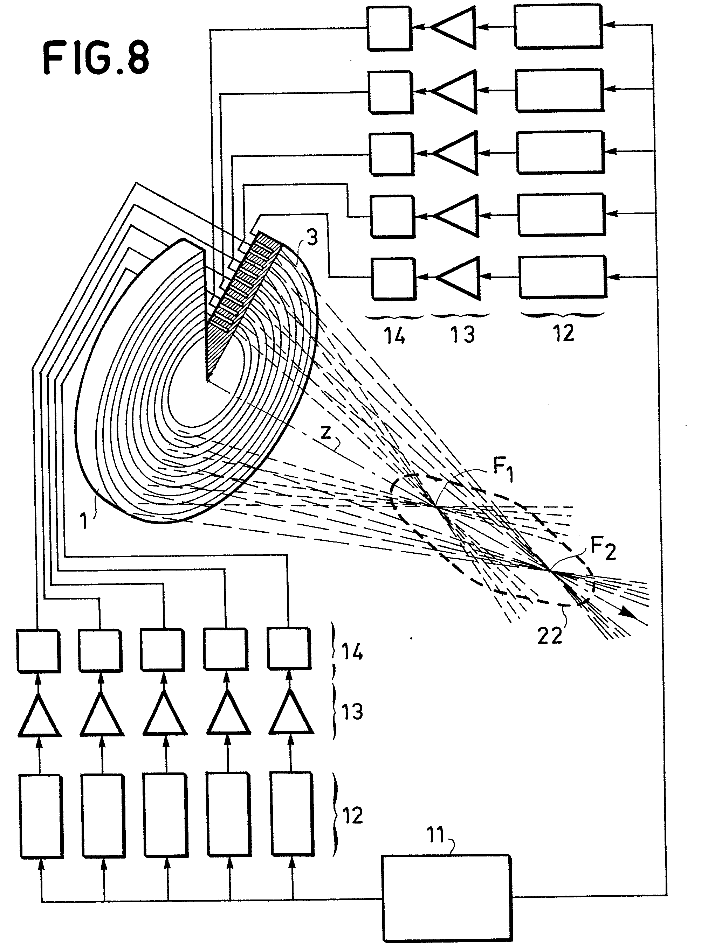

Sur la figure 8, on peut voir un transducteur électromécanique composé d'une plaque piézoélectrique 1 ayant deux faces principales planes revêtues d'électrodes d'excitation. La face avant 3 est recouverte complètement par une électrode de masse. La face arrière visible sur la figure 8 à travers le secteur enlevé comporte une dizaine d'électrodes annulaires concentriques situées entre des tranchées annulaires pratiquées dans la plaque piézoélectrique 1. L'espacement des tranchées est régulier, afin de délimiter un ensemble de transducteurs concentriques fonctionnant tous en demi-onde à la fréquence d'excitation choisie pour l'émission ultrasonore. Les zones émissives élémentaires sont matérialisées sur la figure 8 par des cercles tracés à l'aplomb des tranchées, mais ces lignes de démarcation n'existent que pour les besoins de la compréhension, car la face rayonnante 3 est lisse.

L'excitation électrique du transducteur de la figure 8 est assurée à partir d'un générateur 11 qui délivre une tension alternative de fréquence prédéterminée. La liaison entre ce générateur 11 et les électrodes de la face arrière du transducteur 1 est assurée par deux groupes de cinq voies qui sont reliées respectivement aux électrodes de rang pair et aux électrodes de rang impair. Chaque voie comprend un circuit déphaseur réglable 12, un amplificateur 13 à gain réglable et un circuit adaptateur d'impédance 14. La face arrière du transducteur 1 est couplée à un milieu d'impédance acoustique faible qui par réflexion d'onde assure un rayonnement maximal par la face 3 du transducteur. Cette face 3 est couplée aux tissus biologiques à insonifier par une poche d'eau non représentée qui n'a aucune action réfringente marquée sur les pinceaux ultrasonores émis par la face 3.

Le groupe d'excitation 12-13-14 représenté dans la partie inférieure gauche de la figure 8 est ajusté en amplitude et en phase de façon que les première, troisième, cinquième, septième et neuvième zones annulaires rayonnantes comptées à partir du centre projettent au foyer $F_1$ un premier

rayonnement ultrasonore. Le groupe d'excitation 12-13-14 représenté dans la partie supérieure droite de la figure 8 est ajusté en amplitude et en phase de façon que les seconde, quatrième, sixième, huitième et dixième zones annulaires rayonnantes projettent en un second foyer $F_2$ un second rayonnement ultrasonore. Ces foyers occupent des positions distinctes sur l'axe Z de façon à échauffer uniformément un volume tumoral 22 allongé dans la direction de cet axe.

On peut sans s'écarter du domaine de l'invention, prévoir plus de deux groupements de zones rayonnantes pour obtenir plus de deux foyers le long de l'axe Z. Certains foyers peuvent être situés en dehors de l'axe Z, afin de mieux égaliser l'échauffement du volume tumoral 22.

On remarque sur la figure 8 que l'ouverture angulaire du faisceau convergent au foyer $F_1$ est supérieure à celle du faisceau qui converge au foyer $F_2$. Par contre, les puissances qui atteignent ces deux foyers sont sensiblement les mêmes puisque ces puissances proviennent de surfaces similaires à peu de chose près. En tenant compte des pertes, de la diffraction et de la profondeur de champ, on voit que l'échauffement est plus localisé et plus intense au foyer $F_1$ qu'au foyer $F_2$.

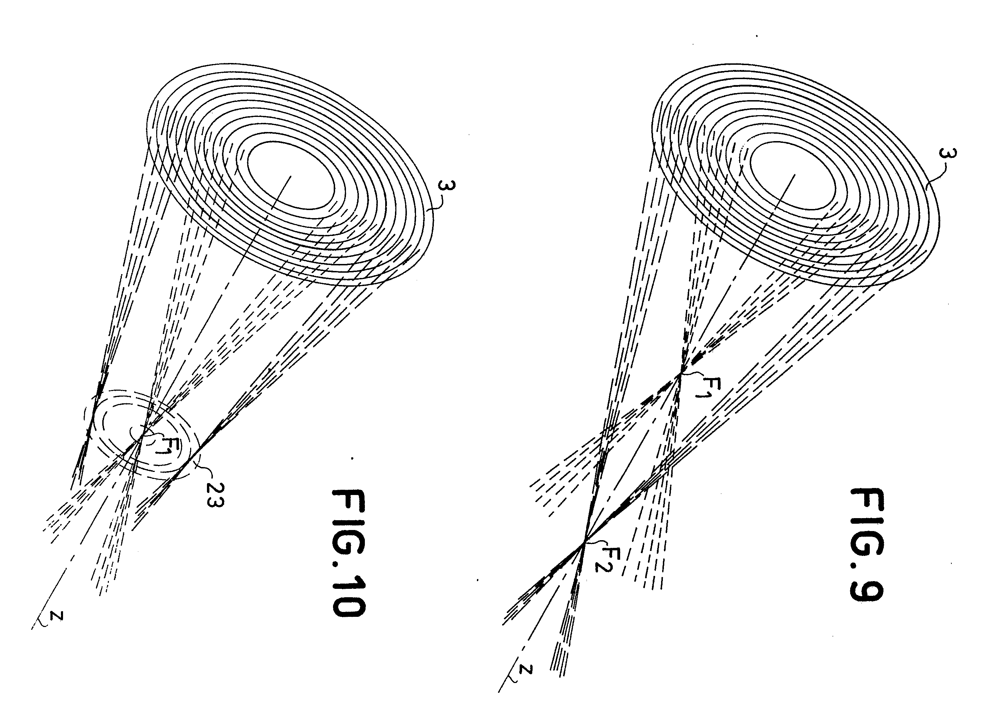

La figure 9 illustre une variante de réalisation du dispositif selon l'invention en se limitant à l'essentiel, c'est-à-dire aux zones annulaires rayonnantes de la face 2 du transducteur et aux pinceaux ultrasonores dont l'addition cohérente a lieu aux foyers $F_1$ et $F_2$. Les excitations fournies par les deux groupes de la figure 8 sont appliquées respectivement aux cinq électrodes contigues les plus proches du centre et aux cinq électrodes contigues les plus éloignées du centre. Ainsi, la puissance émise vers le foyer $F_1$ le plus proche est moindre que celle émise vers le foyer $F_2$. Par contre, les ouvertures angulaires des deux faisceaux projetés peuvent être ajustées pour produire des échauffements d'égales intensités en tablant sur la diffraction, les pertes et la profondeur de champ. Comme les deux émissions ne sont pas imbriquées mais séparées en deux zones bien distinctes, le découplage est amélioré.

En changeant la loi de phase qui s'applique à l'excitation des cinq zones annulaires périphériques de la figure 9, on peut faire en sorte que le foyer ponctuel $F_2$ soit un foyer 23 en forme de couronne qui peut entourer le

foyer $F_1$. Cette variante est illustrée sur la figure 10. On comprend que cette disposition d'un foyer ponctuel cerné par un foyer annulaire permet d'irradier un volume tumoral aplati dans la direction de l'axe Z.

D'une façon générale, le dispositif décrit dans la présente demande en relation avec les figures 8 à 10 offre une grande souplesse grâce aux combinaisons que l'on peut réaliser avec des projections simultanées à foyers ponctuels ou annulaires.

Jusqu'ici on a considéré que les deux groupes d'excitation 12, 13 et 14 sont alimentés par le même générateur 11. Cette solution fait intervenir une seule fréquence d'émission ultrasonore de sorte que les faisceaux peuvent créer par interaction une inhomogénéité due aux franges d'interférences fixes. Pour pallier cet inconvénient, on prévoit d'alimenter les groupes d'excitation 12, 13, 14 de la figure 8 avec des signaux alternatifs ayant des fréquences légèrement différenciées, ces signaux étant produits par deux oscillateurs décalés en fréquence contenus dans le générateur 11. Le décalage en fréquence peut être faible tout en assurant une mobilité suffisante des franges pour homogénéifier l'insonification. Le fonctionnement en demi-onde des éléments rayonnants peut être assuré dans de bonnes conditions à partir d'une plaque piézoélectrique d'épaisseur constante, car l'accord précis peut être ajusté finement en jouant sur la largeur des tranchées annulaires pratiquées au dos de la plaque pour délimiter les éléments rayonnants.

14

## REVENDICATIONS

1. Dispositif d'échauffement localisé de tissus biologiques par projection de rayonnement ultrasonore au moyen d'un transducteur électromécanique associé à un générateur électrique d'excitation ayant une fréquence d'oscillation prédéterminée, caractérisé en ce que ledit transducteur est du type demi-onde et comporte une plaque plane de matériau piézoélectrique (1) dont une première face (2) comporte un réseau de sillons annulaires (4) concentriques ayant un pas régulier (p) voisin de la longueur d'onde dans l'eau dudit rayonnement ; la seconde face (3) de ladite plaque (1) étant lisse ; les zones rayonnantes annulaires de ladite plaque (1) encadrées par lesdits sillons (4) étant munies d'électrodes en contact avec lesdites faces (2, 3) ; ledit générateur électrique comportant un ensemble de moyens déphaseurs réglables (12) destinés à alimenter au moins partiellement lesdites électrodes par des tensions dont les phases sont ajustables pour focaliser ledit rayonnement (9) au sein des tissus biologiques (8) couplés acoustiquement à ladite seconde face (3).

2. Dispositif selon la revendication 1, caractérisé en ce que ladite seconde face (3) est coiffée par une poche (5, 6), perméable audit rayonnement et remplie d'un liquide (15) ayant une impédance acoustique voisine de celle de l'eau.

3. Dispositif selon l'une quelconque des revendications 1 et 2, caractérisé en ce que la face de ladite plaque (1) comportant lesdits sillons (4) est coiffé d'un couvercle (7) renfermant un milieu d'impédance acoustique négligeable par rapport à l'impédance acoustique dudit matériau piézoélectrique (1).

4. Dispositif selon l'une quelconque des revendications 1 à 3, caractérisé en ce que la plaque (1) comporte en son centre (10) des moyens transducteurs ultrasonores (24, 27) coopérant avec un système de visualisation (29, 30) destiné à surveiller la zone irradiée et à contrôler l'échauffement ; les moyens de visualisation mettant en oeuvre l'émission et la réception de rayonnements ultrasonores de fréquence supérieure à ladite fréquence prédéterminée.

5. Dispositif selon l'une quelconque des revendications 1 à 4, caractérisé en ce que lesdites zones rayonnantes annulaires rayonnent par secteurs (24, 25, 26) avec des excitations électriques déphasées permettant de réaliser un balayage transversal du faisceau focalisé.

6. Dispositif selon l'une quelconque des revendications 1 à 5, caractérisé en ce que l'excitation électrique desdites zones rayonnantes annulaires est déphasée selon un loi qui focalise le faisceau selon un contour circulaire fermé (23).

7. Dispositif selon l'une quelconque des revendications précédentes, caractérisé en ce que ledit matériau piézoélectrique est une céramique piézoélectrique métallisée sur ses deux faces (2, 3).

8. Dispositif selon la revendication 7, caractérisé en ce que le rapport de la largeur (l) desdites zones rayonnantes annulaires à l'épaisseur (e) de ladite plaque est compris entre 0,5 et 1.

9. Dispositif selon l'une quelconque des revendications précédentes, caractérisé en ce que l'émission du rayonnement ultrasonore est une émission continue.

10. Dispositif selon l'une quelconque des revendications 1 à 8, caractérisé en ce que l'émission du rayonnement ultrasonore est une émission pulsée.

11. Dispositif d'échauffement localisé selon l'une quelconque des revendications précédentes, caractérisé en ce que les zones rayonnantes d'une même plaque (1) forment N groupes émetteurs excités par des tensions dont les phases assurent la projection simultanée de N rayonnements ultrasonores vers N foyers distincts ($F_1$, $F_2$, 23). ·

12. Dispositif selon la revendiation 11, caractérisé en ce que les phases desdites tensions sont choisies pour que l'un au moins des N rayonnements ultrasonores aboutisse à un foyer ponctuel ($F_1$, $F_2$).

13. Dispositif selon la revendication 11, caractérisé en ce que les phases desdites tensions sont choisies pour que l'un au moins des N rayonnements ultrasonores aboutise à un foyer annulaire (23).

14. Dispositif selon l'une quelconque des revendications 11 à 13, caractérisé en ce que les fréquences desdits rayonnements ultrasonores sont différenciées.

15. Dispositif selon l'une quelconque des revendications 11 à 14, caractérisé en ce que les rayonnements ultrasonores sont issus de zones annulaires rayonnantes imbriquées.

16. Dispositif selon l'une quelconque des revendications 11 à 14, caractérisé en ce que l'un au moins des rayonnements ultrasonores est issu d'une plage émissive comportant plusieurs zones annulaires contigues.

FIG.1

FIG. 2

FIG.3

FIG.4

FIG.5

FIG.6

0068961

(a)

Z

(b)

**FIG.7**

FIG.8

FIG.9

FIG.10